# EUROPEAN PATENT APPLICATION

(11) **EP 3 708 003 A1**
(43) Date of publication of application: **16.09.2020**
(21) Application number: 20162118.2
(22) Date of filing: 10.03.2020
(51) Int. Cl.: A01N 63/30, C12N 1/14, C12R 1/645, A01P 7/04

(54) **THE BEAUVERIA BASSIANA ENTOMOPATHOGENIC FUNGAL SPECIES, METHOD OF ITS APPLICATION AND PREPARATION CONTAINING THE SPECIES PORES**

(30) Priority: 12.03.2019 CZ 20190141
(71) Applicant: Sweetcakes s.r.o., 12000 Praha (CZ)
(72) Inventor: Bohatá, Andrea, 31800 Plzen (CZ)
(74) Representative: Sedlák, Jirí

(57) **Abstract**

The strain of entomopathogenic fungus *Beauveria bassiana* for the protection of forest stands deposited under the sample number CCM 8382 in the Czech Collection of Microorganism can be used for the biological control of forest stands against the populations of pests belonging to the family Scolytinae and the family Molytinae, especially the large pine weevil.

## Description

### Field of the Invention

The invention concerns of the strain of entomopathogenic fungus *Beauveria bassiana* for the protection of forest stands against the populations of their pests, the method of protection of forest stands using the entomopathogenic fungus and the product containing spores of entomopathogenic fungus.

### Background of the Invention

The forest stands in the territory of the Czech Republic have been facing bark beetle calamities caused by numerous populations of pests from the family Scolytinae (bark beetles). Due to the mass outbreak of bark beetles, also healthy trees that would have chance to resist infestation under standard conditions are heavily infested. The long-time dry periods have reduced the defence capability of the trees, which resulted in a huge increase of the bark beetle populations and damage to forest stands. Then on the timber felling and newly planted areas, seedlings are damaged by the large pine weevil *Hylobius abietis* (family Molytinae) making small, perpendicular or funnel-shaped hollow often reaching as far as the superficial layer of wood. In the case of heavy damage, the pits aggregate along the perimeter of the tiny woody stem and the seedling dies.

One of the possible approaches to the control of forest pests damaging forest stands in the case of bark beetle is infested trees debarking, active cutting of infested trees, transport of infested trees outside the forest stands and destruction of the pests larvae prior to their metamorphosis into adults capable of reproduction. Due to the intensive exploitation, the market is now saturated by wood sold at low purchase price, which results in insufficient motivation to further process wood from the infested forest stands.

An alternative to logging and mechanical treatment of trees are chemical substances. For the monitoring and trapping of bark beetles, pheromone traps are used where adults are lured into pheromone-baited traps. When adults enter the trap, they are captured in the catching container from where they are not able to escape. Another protective measure employed to control the population of adults are trap trees being felled and covered by branches to prevent drying. The trap trees are attractive for adults and are used for their entrapment. After infestation, the trap trees are chemically treated with insecticides that kill the adults which are under the bark. Alternatively, the trap trees are removed from the forest stand before the bark beetle finish its development cycle and are treated again with pesticides or debarking outside the forest stand. Decontamination of the trap trees is performed by spraying or by the application of powdered insecticides that reduce the populations of bark beetles immediately after application.

Among the disadvantages of the use of chemical substances is the fact that the artificially developed substances are toxic not only to the forests pests but also to other animal species, in particular not-target insects. Moreover, there is a risk of leakage of the chemical substances that in concentrated form may be harmful effects on the environment.

The disadvantages of chemical solutions are being replaced by the quickly developing biological methods where a pathogenic micro-organism is isolated, e.g. fungal species, invading and infecting the pest populations, is isolated. An example of such an invention is the entomopathogenic fungus disclosed in the CZ 300 701 B6 document. The invention discloses a new strain CCM 8367 of entomopathogenic fungus *Isaria fumosorosea* that effectively invades the pests belonging to the Lepidoptera, Coleoptera, Hymenoptera, and Homoptera orders. The advantage of the biological control is that its concerns micro-organisms naturally occurs in the environment, which means that their artificially increased presence in the environment does not pose a threat to other animal species except of their natural hosts. Once the population of natural hosts is reduced, the pathogenic micro-organism loses the opportunity for reproduction and its occurrence in the environment start decreasing.

The task of the invention is the protection of forest stands against forest pests using the strain of entomopathogenic fungus *Beauveria bassiana* that would invade and infect the populations of forest pests belonging to the family Scolytinae and family Molytinae with no negative impact on other animal and plant species. And also, that the strain has high viability and efficiency and that could be processed by industrial methods into products suitable for large-scale application.

### Summary of the Invention

The set task has been resolved by the strain of entomopathogenic fungus *Beauveria bassiana* for the protection of forest stands deposited under the number CCM 8382 in the Czech Collection of Microorganisms.

The advantages of the entomopathogenic fungus according to the invention rest in the fact that the fungus *Beauveria bassiana* is naturally occurred in forest stands of the temperate zone to which the forest stands in the Czech Republic belong. This means that the new strain of fungus according to the invention is not an exotic invasive strain artificially implanted into the forest stand environment but naturally co-existing with the other fungi and also plant and animal species. Another advantage is that the strain CCM 8382 is virulent to the pests (bark beetles) belonging to the family Scolytinae that cause the bark beetle calamities in forest stands, and belonging to the family Molytinae, in particular the large pine weevil that cause damage to seedlings. The last but not the least advantage rests in the fact that the strain CCM 8382 can be easily produced under controlled conditions using liquid state fermentation as well as solid state fermentation, which makes the strain suitable for large-scale industrial production and for large-scale application to control the bark-beetle calamities in forest stands.

The invention also comprises the method of application of the entomopathogenic fungus for the protection of forest stands, the essence of which rests in the fact that reduction of the populations of forest pests belonging to the family Scolytinae and to the family Molytinae, in particular the large pine weevil, is attained by the application of the strain CCM 8382 of entomopathogenic fungus *Beauveria bassiana.* The strain CCM 8382 produce spores that attach to the surface of plants and tree trunks where they can passively wait for a contact with the pest whom they consequently infect. If spores attach on the body of an individual from the family Scolytinae and from the family Molytinae, then after the spores have germinated, the fungus hyphae penetrate into the individual's body cavity where the blastospores (budding spores) of the fungus start forming. In the following phase of development, the pests is killed by fungus which produced in the hemolymph dense mass of blastospores. In the final phase of development, the fungus hyphae proliferate onto the surface of the killed host. The dead host becomes covered by the white mycelium and fungus sporulate, i.e. fungus produces a new generation of the spores. The new spores spread in the environment and infect other individuals in the pest populations. Thus, a new cycle of reproduction is started on a new host.

In a preferred embodiment of the method according to the invention, the spores of fungal strain CCM 8382 will be used for the protection of forest stands and will be applied in the form of liquid spray and/or dispersed powder. Distribution of the spores is possible using the wet or dry process and the selection of the method depends on the conditions present in the site of the forest stand concerned. The wet process in the form of suspension spraying is suitable for both the seedlings' protection of forest stand, and for preventive or curative treatment of a forest stand. The dry process in the form of cold smoke (dusting) is suitable in particular for preventive and curative treatment of a forest stand.

An integral part of the invention is the product for the protection of forest stands, which contain the spores of entomopathogenic fungus dispersed in the nutritive carrier.

The essence of the invention rests in the fact that the product ("spore concentrate") comprises spores of the fungus *Beauveria bassiana* strain CCM 8382 in the concentration being either 10⁹ or 10¹⁰ spores in 1 g of the nutritive carrier. The advantage of the spore concentrate is undoubtedly the high content of infectious spores in 1 g of the product. Another advantage of the space-saving of the spore concentrate is a possibility to prepare the final product in the required volume "in situ" sufficiently in advance or immediately prior to application. In addition, the product in the form of spore concentrate can be stored at a temperature of 4 °C for a long time, up to one year, which is useful as the necessity to store large volumes of the final product at low temperatures is eliminated.

The spore concentrate according to the invention is the basis for preparation of suspension intended for liquid spraying. By suitable selection of the volume of the liquids, it is possible to dilute the preparation to attain the spore concentration corresponding to the intensity of the forest stand infestation by bark beetle. Therefore, the suspension comprises water, the nutritive carrier and spores of the fungus *Beauveria bassiana,* strain CCM 8382 with the concentration from 10⁶ to 10⁷ spores in 1 ml of the suspension. In another preferred embodiment, the suspension may also comprise some of vegetable oils as an additive. The application of spores in the liquid suspension is ideal for spraying forest stands where droplets of the suspension can run down from the tree tops (crown) towards the lower parts of the trees.

In addition, a powder mixture comprising finely ground diatomaceous earth, the nutritive component and spores of the fungus *Beauveria bassiana,* strain CCM 8382 can be prepared from the spore concentrate according to the invention. By a good choice of the quantity of finely ground diatomaceous earth, it is possible to reduce the concentration of the spores to correspond to the degree of infestation of the forest stand by harmful pests. Thus, the final powder mixture can contain the concentration of spores of the fungal species ranging from 10⁸ to 10⁹ in 1 g of the mixture. In addition, distribution of spores by powder dispersion, in particular in the form of cold smoke dusting, is suitable in sites with higher trees in the forest stand where the powder is drifted due to air circulation up to the tree crowns and is dispersed onto larger areas of the forest stand.

The invention is intended for the protection of forest stands using a biological control that is, based on available references, friendly to the other forms of life found in the given site except for the target groups of pests. In the case of destruction of the populations of the pests of families Scolytinae and Molytinae, the fungus strain will start degrading naturally as the environment comprises organisms that are feeding on the fungi. Nevertheless, some quantity will survive in nature as it is the fungus natural environment. In addition, the strain is able to survive at lower temperatures, which means that it is able to overwinter in the environment and in the case of a new invasion of the pests, it can start its natural expansion without control by humans. The fungal strain is also suitable for industrial production of spores to produce the product where the spores can survive on the nutritive carrier at a stable temperature of 4 °C over a period of up to one year.

### Explanation of Drawings

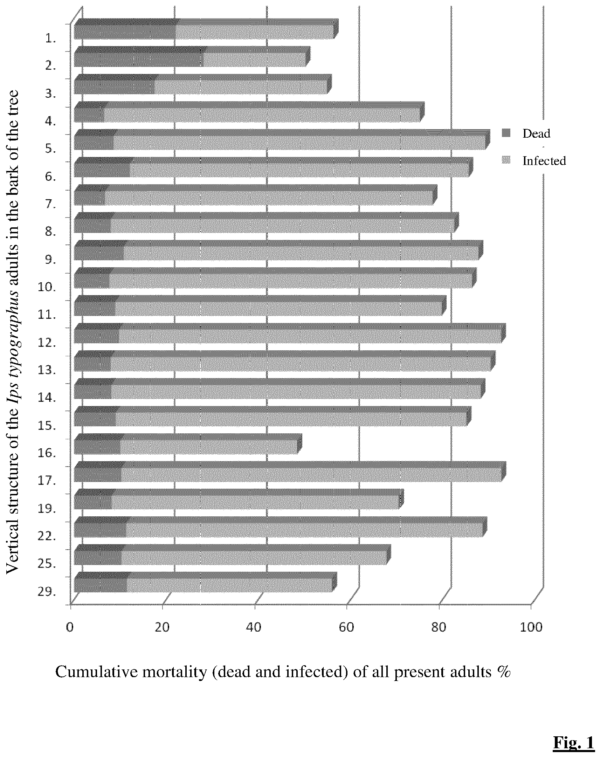

The present invention will be explained in detail by means of the following figures where Fig. 1 is a graph of the vertical structure of the population of the *Ips typographus* adults in the bark of a tree for the purposes of Example 3 of the invention embodiment.

### An Example of the Invention Embodiment

It shall be understood that the specific cases of the invention embodiments described and demonstrate below are provided for illustration only and do not limit the invention to the examples provided here. Specialists skilled in the field will find or, based on routine experiments, will be able to provide a greater or lesser number of equivalents to the specific embodiments of the invention which are described here.

The spore biomass of the strain CCM 8382 of fungus *Beauveria bassiana* is produced by solid state fermentation on a natural substrate. When the maximum sporulation is attained, in the first phase of formulation, spores are transferred from the substrate onto the nutritive carrier and the product is stored in the form of a spore concentrate (10⁹ or 10¹⁰ spores in 1 g) at a temperature of approximately 4 °C.

Before distribution, the spore concentrate is mixed with inert powder (finely ground diatomaceous earth, powder form) in the ratio of 1:100 (= 1.0 × 10⁸ spores in 1 g) (preventive application of the invention) or in the ratio of 1:10 (= 1.0 × 10⁹ spores in 1 g) (curative application of the invention) and the final product is weighed into transport plastic packages of 1,000 g. Every package is equipped with an identification label specifying (among other information) the number of the entomopathogenic fungus and the site for which the preparation is intended.

Alternativaly, the concentrate is mixed with water with/without added vegetable oil immediately prior to application and the resulting suspension or emulsion is then applied onto forest stands.

After test applications, no killing of natural enemies of pests or other beneficial insects or mites has been observed.

In the under the bark niche, the presence of the strain CCM 8382 was observed only on the bark beetle species of the family Scolytinae.

### Example 1: Application of the conidial suspension of the strain CCM 8382 of fungus B. bassiana to treat the adults of bark beetle (Ips typographus) in laboratory conditions

The surface of fully sporulated strain CCM 8382 on a natural substrate was poured over by sterile solution of distilled water with added detergent 0.05% Tween 80. The concentration of conidia in the prepared suspension was calculated using the haemocytometer (improved Neubauer counting chamber) and subsequently adjusted to the standard titre of 1.0 × 10⁷ conidia in 1 ml of the suspension.

Prior to the experiment, wet chamber were prepared. The wet cell comprised a sterile, disposable plastic box of the size of 90 × 70 × 60 mm with wetted sterile cellulose at the bottom.

Adults of the bark beetle were obtained from an infested tree where pieces of spruce bark comprising adults were collected in the wood and in addition, crushed bark with present adults of bark beetle was added. In the laboratory conditions, adults from the crushed bark and spruce bark were separated and put into the sterile plastic box.

A total of 100 adults treated with the strain suspension and 100 adults treated with distilled water containing detergent (control) were used in the experiment.

During treatment, each adult was individually dipped in the prepared strain suspension for 2 seconds and after the treatment, adult was put onto filter paper to remove excessive suspension of the fungus. The 20 adults treated by the strain suspension in five repetitions were inserted into each wet cell. In the control variant, adults were individually dipped into distilled water containing detergent 0.05% Tween 80 and after excessive water was removed, the adults were put into wet cells as well. The control variant had also five repetitions.

The plastic boxes with the treated as well as control adults were put into the thermostat and incubation was performed in dark at the temperature of 21 °C. Evaluations were made after 3, 5, and 7 days following the treatment. Within the framework of the treated variant and the control, the condition of the adults was recorded and classified into one of the following categories: "alive - dead - infected". The cumulative mortality expresses the sum of dead and infected adults.

The structure of the population of bark beetle adults after the treatment by the strain suspension:

| Variant | Day | alive (%) | dead (%) | infected (%) | cumulative mortality |
|---|---|---|---|---|---|
| Control | Day 3 | 94.20% | 5.80% | 0.00% | 5.80% |
| | Day 5 | 86.60% | 13.40% | 0.00% | 13.40% |
| | Day 7 | 81.90% | 18.10% | 0.00% | 18.10% |
| CCM 8382 | Day 3 | 54.20% | 34.40% | 11.40% | 45.80% |
| | Day 5 | 6.10% | 13.90% | 80.00% | 93.90% |
| | Day 7 | 0.80% | 6.40% | 92.80% | 99.20% |

### Example 2: Application of the powder formulation of the strain CCM 8382 of the fungus B. bassiana to treat the bark beetle (Ips typographus) adults in laboratory conditions

Conidia from the fully sporulated strain CCM 8382 on a natural substrate were mixed with an inert carrier after drying. In the powder formulation prepared in this manner, the concentration of conidia in 1 g was determined. Subsequently, the powder mixture was adjusted using the inert carrier preparation to the standard titre of 1.0 × 10⁷ conidia in 1 g of the powder formulation.

Prior to the experiment, wet chamber were prepared again. Adults obtained from the spruce forest stand were used for the experiment.

In this experiment, the powder formulation of the strain CCM 8382 was applied in a thin layer on the bottom of a Petri dish. The adults were put onto the powder formulation where they moved for 30 seconds. Then the adults were placed to the wet chamber, 20 adults in five repetitions. In the control variant, the adults were exposed only to the inert carrier without the presence of fungus conidia. Again, five repetitions of 20 adults were prepared in the control variant.

A total of 100 adults were exposed to the powder formulation of the strain and 100 adults were exposed to the inert carrier. The plastic boxes with the treated as well as control adults were put into the thermostat and incubation was performed in dark at the temperature of 21 °C. Evaluations were made after 3, 5, and 7 days following the treatment. Within the framework of the treated variant and the control, the condition of the adults was recorded and classified into one of the following categories: "alive - dead - infected". The cumulative mortality expresses the sum of dead and infected adults.

The structure of the population of bark beetle adults after exposure to the powder formulation:

| Variant | Day | alive (%) | dead (%) | infected (%) | cumulative mortality |
|---|---|---|---|---|---|
| Control | Day 3 | 94.80 % | 5.20% | 0.00% | 5.20% |
| | Day 5 | 88.30% | 11.70% | 0.00% | 11.70% |
| | Day 7 | 82.90% | 17.10% | 0.00% | 17.10% |
| CCM 8382 | Day 3 | 47.40% | 36.20% | 16.40% | 52.60% |
| | Day 5 | 3.10% | 10.60% | 86.30% | 96.90% |
| | Day 7 | 0.00% | 3.20% | 96.80% | 100.00% |

### Example 3: Application of the powder formulation of the strain CCM 8382 of the fungus B. bassiana for the treatment of forest stands against bark beetles

The strain CCM 8382 was produced by large-volume solid state fermentation on a natural substrate. After drying, conidia were harvested into the nutritive carrier and adjusted to the standard titre of 1 × 10¹⁰ in 1 g of the spore concentrate. The spore concentrate was then diluted by the inert carrier to attain the conidia concentration of 1 × 10⁸ in 1 g of the powder mixture.

The powder formulation was applied in a forest in the form of cold smoke (dusting) to the forest stand among which trees infested by bark beetles were present. The cold dust smoke was carried by air up to the tree crowns. Application was performed at the time when bark beetles were swarming. Eight weeks after application, a tree selected at random was cut. A detailed analysis of the randomly selected tree revealed that infection by the strain CCM 8382 of the fungus *B. bassiana* is evenly dispersed along the entire tree, namely including the parts in the tree crown and at the top of the trunk, i.e. at a height of approximately 21 m.

The results of the experiment focused on the verification of effects of the fungus *Beauveria bassiana* applied in the form of spore powder formulation were contributed by a whole of the tree. The tree was cut into blocks which were divided lengthwise into three groups. Then, in three observation days with a monthly interval, one third of the blocks was cut into pieces and cumulative mortality (dead and infected) of all present adults, including those hidden in the bark, was recorded. Within the framework of the evaluation, the condition of the adults was recorded and each adult classified into one of the following categories: "alive - dead - infected". After all adults have been taken out, alive adults were put into the wet chamber to ascertain whether the infection would break out in them under optimal conditions.

The first analysis was performed 8 weeks after the application of the powder formulation, the second analysis 12 weeks after application and the third analysis 16 weeks after the application. The first analysis proved that cumulative mortality in the first third of the blocks in the vertical position of the tree ranged between 23.3% and 60.8%. During the second analysis cumulative mortality based on the second third of the blocks was ascertained between 20.4 and 44.0%. For the first two analyses, the results are provided without the follow-up incubation of alive and dead individuals. For the third analysis, cumulative mortality on the remaining blocks ranged from 30.6 to 52.6% without incubation of alive individuals. After the incubation of alive adults, cumulative mortality increased from 48.4 to 92.7%. By means of incubation, it has been proven that the fungus spores remain attached on alive adults and if suitable conditions occur, they are able to infect also these individuals.

The graph in Figure 1 provides the vertical structure of the *Ips typographus* adults in the bark of the tree following the third analysis.

### Industrial Applicability

The strain CCM 8382 of entomopathogenic fungus *Beauveria bassiana,* the method of its application and the preparation containing its spores according to the invention can be employed within the framework of biological control of forest stands against the bark beetle calamities and will become one of the methods of the "Forest Integrated Management".

## Claims

1. The strain of entomopathogenic fungus *Beauveria bassiana* for the protection of forest stands deposited under the CCM 8382 sample number in the Czech Collection of Microorganisms administered by the Faculty of Science of the Masaryk University, Kamenice 753/5, 625 00 Brno.

2. The method of application of the entomopathogenic fungus for the protection of forest stands, **characterized in that** the strain CCM 8382 of entomopathogenic fungus *Beauveria bassiana* is used for reduction of pests populations forest stands belonging to the family *Scolytinae* and the family *Molytinae,* specifically the large pine weevil.

3. The method according to claim 2, **characterized in that** the forest stand is treated with spores of the fungus *Beauveria bassiana,* strain CCM 8382.

4. The method according to claim 3, **characterized in that** the spores are applied in the form of liquid spraying and/or dispersed powder.

5. The method according to claim 4, **characterized in that** the application by liquid spraying is used for the protection of seedlings forest stand.

6. The method according to claim 4, **characterized in that** the application by dispersed powder is used for the preventive as well as curative treatment of forest stands.

7. The product for the protection of forest stands comprising spores of the entomopathogenic fungus dispersed in the nutritive carrier, **characterized in that** it comprises spores of the fungus *Beauveria bassiana* the strain CCM 8382 in the concentration ranging from 10⁹ to 10¹⁰ spores in 1 g of the nutritive carrier.

8. The product according to claim 7, **characterized in that** it is in the form of suspension for liquid spraying consisting of water, the nutritive carrier and spores of the fungus *Beauveria bassiana* strain CCM 8382 in the concentration ranging from 10⁶ to 10⁷ spores in 1 g of the suspension.

9. The product according to claim 8, **characterized in that** the suspension comprises at least one vegetable oil as an additive.

10. The preparation according to claim 7, **characterized in that** it is in the form of a powder mixture consisting of finely ground diatomaceous earth, the nutritive component and spores of the fungus *Beauveria bassiana* strain CCM 8382 in the concentration ranging from 10⁸ to 10⁹ spores in 1 g of the powder mixture.
